(19) Europäisches Patentamt
European Patent Office
Office européen des brevets

(11) Numéro de publication : **0 442 783 A1**

(12) # DEMANDE DE BREVET EUROPEEN

(21) Numéro de dépôt : **91400294.4**

(22) Date de dépôt : **07.02.91**

(51) Int. Cl.[5] : **C07H 7/02**, C12P 19/02, A23K 1/17

Le microorganisme a été déposé auprès de Centraal Bureau voor Schimmelcultures Baarn sous le numéro CBS 473-89.

(30) Priorité : **08.02.90 FR 9001442**

(43) Date de publication de la demande : **21.08.91 Bulletin 91/34**

(84) Etats contractants désignés : **AT BE CH DE DK ES FR GB GR IT LI LU NL SE**

(71) Demandeur : **RHONE-POULENC NUTRITION ANIMALE**
**Rue Marcel Lingot**
**F-03600 Commentry (FR)**

(72) Inventeur : **Margraff, Rodolphe**
**16 Allée du Potager**
**F-91170 Viry-Chatillon (FR)**
Inventeur : **Kiener, Thierry**
**1 rue des Fleurs**
**F-03310 Neris les Bains (FR)**
Inventeur : **Kies, Arie**
**1 rue Daniel**
**F-03600 Commentry (FR)**

(74) Mandataire : **Le Pennec, Magali et al**
**RHONE-POULENC RORER SA, Direction des Brevets, 20 Avenue Raymond Aron**
**F-92165 Antony Cédex (FR)**

(54) **Nouvelle elfamycine, son procédé de préparation et son utilisation.**

(57) La présente invention concerne un nouveau dérivé de la famille des elfamycines de formule (II), son procédé de préparation et son utilisation dans l'alimentation animale des animaux monogastriques.

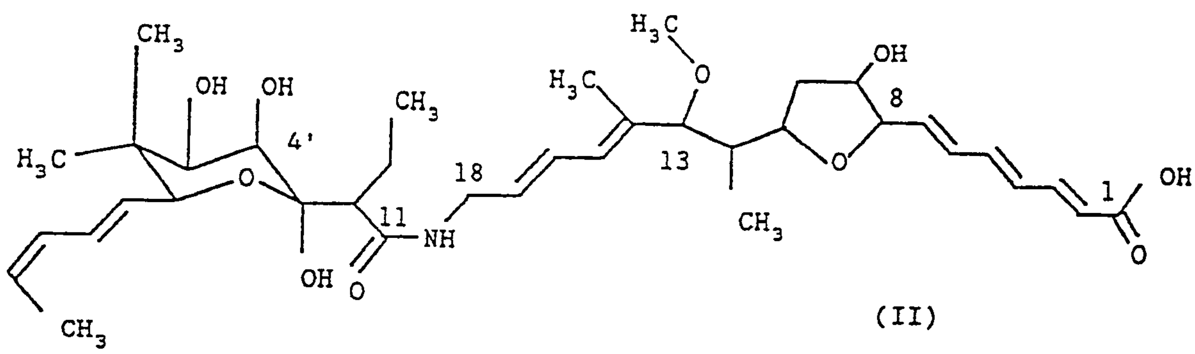

(II)

EP 0 442 783 A1

# NOUVELLE ELFAMYCINE, SON PROCEDE DE PREPARATION ET SON UTILISATION

La présente invention concerne un nouveau composé de la famille des elfamycines, son procédé de préparation et son utilisation.

Les elfamycines sont des antibiotiques connus depuis longtemps pour le traitement des infections humaines et animales.

La plupart des elfamycines connues à ce jour répondent à la formule générale suivante :

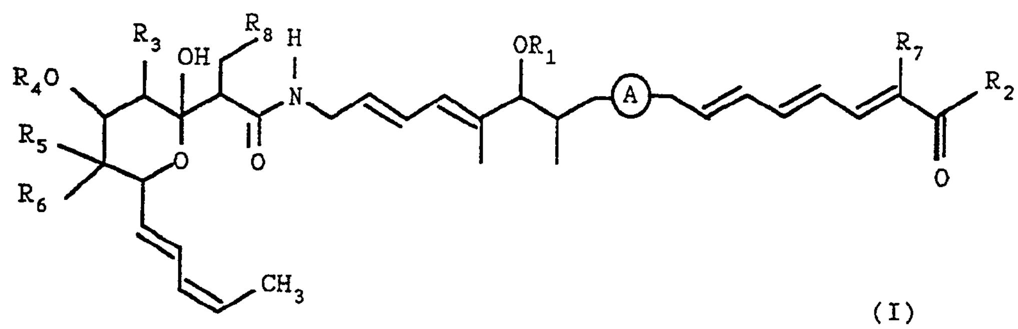

(I)

dans laquelle :

– le groupe $R_1$ représente un motif alkyle,

– le groupe $R_2$ représente un groupe hydroxyl ou un radical pyridone substitué,

– les groupes $R_3$, $R_5$, $R_6$, $R_7$ représentent soit des groupes méthyl, soit hydroxy, soit hydrogène, soit alkyl

– le groupe $R_4$ représente un groupe alkyl, hydrogène, glycosidyl,

– le groupe $R_8$ représente un atome d'hydrogène ou un groupe O(diginose)$_2$

– le groupe A représente une liaison dihydroxyéthyldiyl ou un cycle mono ou dihydroxyfuranique en position 2,5 du cycle furanique.

Ces elfamycines peuvent être divisées en 3 classes selon la nature des radicaux $R_2$ et $R_4$.

La première classe est limitée aux composés de formule (I) pour lesquels $R_2$ = OH et A représente un groupe hydroxyfuranique.

Dans cette classe, on peut citer les brevets US 4476139, US 4705688 et US 4753798 qui concernent des composés de formule (I) ($R_1$=$CH_3$, $R_2$=OH, $R_3$=$R_4$=$R_7$=$R_8$=H, $R_5$=$CH_3$, $R_6$= OH) pour le premier brevet et des composés de formule (I) ($R_1$=$CH_3$, $R_2$=OH, $R_3$=$OCOCH_2C_6H_5$ ou OH, $R_4$=H ou $COCH_2C_6H_5$, $R_5$=$R_6$=$CH_3$, $R_5$=$R_6$=$CH_3$, $R_7$=H, $R_8$=-O(diginose)$_3$ pour le deuxième et le troisième. Ces composés présentent une activité antibiotique importante vis-à-vis de nombreux germes (Bacillus, Proteus, Staphylococcus, Brucella, Escherichia coli, Pseudomonas, Enterobacter, Streptococcus). Ces elfamycines sont utilisées en thérapeutique contre l'ensemble des infections qui y sont sensibles, mais elles peuvent également être utilisées comme agent favorisant la croissance des animaux monogastriques.

La seconde classe d'elfamycines est constituée principalement par des composés de formule (I) pour lesquels $R_2$ représente un groupe pyridone. Dans cette classe, on peut citer le brevet US 3708577 qui décrit l'utilisation comme agent permettant la croissance des animaux d'elfamycines substituées par un groupe pyridone éventuellement substitué telles que celles notamment vendues sous les dénominations Efrotomycine, Aurodox, Kirromycine, Heneicomycine (MAEHR, H ; M. LEACH ; T.H. WILLIAMS & J.F. BLOUNT ; The Chemistry of Aurodox and related antibiotics (Can. J. Chem. 58, 502-526, 1980). Elles présentent elles aussi un effet antibiotique sur de nombreux germes et favorisent la croissance des animaux.

Par contre, la présence de ce groupe pyridone rend leur extraction des milieux de fermentation plus laborieuse et leur stabilité à l'humidité et à la chaleur plus faible.

Une troisième classe d'elfamycines est constituée des composés de formule (I) dans lesquels le groupe $R_4$ représente un motif polyglycidyl. Ces elfamycines sont notamment décrites dans les brevets US4497969 et US4024251.

Ces elfamycines présentent comme toutes celles citées précédemment un effet antibiotique et un effet sur la croissance des animaux. Certaines d'entre elles sont produites sous la forme de deux isomères dont la séparation est difficile.

Dans l'état actuel de nos connaissances, il semblait nécessaire d'utiliser pour favoriser la croissance des animaux des antibiotiques à large spectre d'activité, ces antibiotiques étant préparés selon des procédés présentant des productivités faibles et selon des techniques d'extraction lourdes. En plus, certains d'entre eux particulièrement ceux des deuxième et troisième classes présentent une stabilité au chauffage insuffisante qui ne permet pas leur intégration dans les granulés utilisés pour l'alimentation des animaux, la granulation étant effectuée par chauffage et sous pression (US 4597969).

L'industrie de l'alimentation animale est toujours à la recherche d'un composé permettant de favoriser la croissance des animaux, facile à produire par des procédés de fermentation ayant des productivités suffisantes, facile à extraire et surtout présentant peu de propriétés antibiotiques.

En effet, depuis quelques années, les législations nationales commencent à introduire certaines réglementations en ce qui concerne l'usage d'antibiotiques dans l'alimentation des animaux destinés à l'alimentation humaine. Certains pays ont même interdit totalement l'usage des antibiotiques pour l'alimentation des animaux.

La présente invention a permis de trouver un nouveau composé de la famille des elfamycines ne présentant pas ou peu d'effet antibiotique mais présentant un effet sur la croissance des animaux de même amplitude que les antibiotiques précédemment décrits.

Le composé de la présente invention répond à la formule (II) :

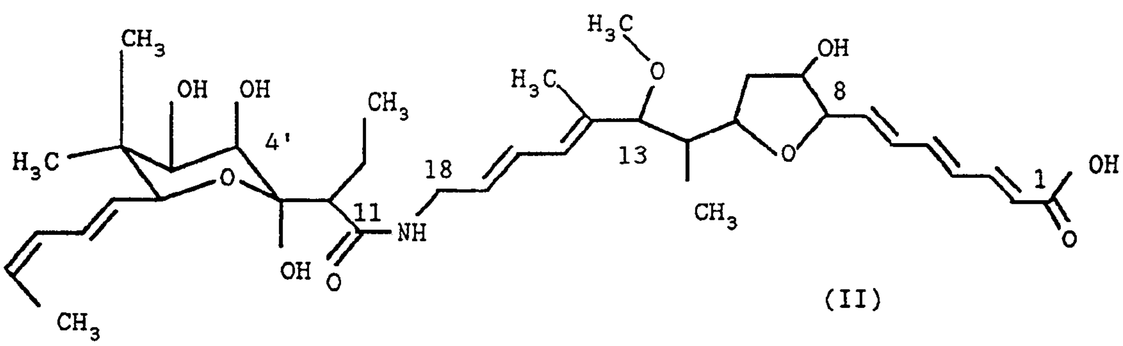

(II)

ou à un de ses sels tels que les sels alcalins (sodium, potassium), les sels alcalino-terreux (calcium), les sels azotés (ammonium, triéthylamine, lysine, arginine, ...).

Le procédé de préparation des composés selon l'invention est un procédé de fermentation à partir d'une souche spéciale du genre des Streptomyces déposée au C.B.S. (Centraal Bureau voor Schimmelcultures, BAARN, NEDERLAND) sous le numéro CBS 473.89.

Cette souche peut être utilisée à l'état sauvage ou après avoir subi des mutations par exemple à l'aide de rayons ou de produits chimiques bien connus de l'homme de l'art. Elle ne produit que le seul composé de formule (II).

Cette souche est mise en culture en tubes sur un milieu gélosé contenant :

| | |
|---|---|
| - amidon soluble : | 10 g/l |
| - phosphate dipotassique : | 1 g/l |
| - sulfate de magnésium : | 1 g/l |
| - chlorure de sodium : | 1 g/l |
| - sulfate d'ammonium : | 2 g/l |
| - carbonate de calcium : | 2 g/l |
| - traces de sel : | 1 g/l |
| - gélose : | 20 g/l |

On stérilise ce milieu et on l'ensemence à partir de la souche de streptomyces C.B.S. 473.89. On maintient 3 à 4 semaines à 26°C.

On prépare dans un second stade un inoculum dans un erlenmeyer de 2 litres contenant un milieu de culture composé de :

| | |
|---|---|
| - peptone : | 10 g/l |
| - extrait de levure : | 5 g/l |
| - glucose : | 10 g/l |
| - chlorure de sodium : | 5 g/l |
| - gélose : | 1,5 g/l |

Ce milieu est ajusté à PH 7,4 et stérilisé. On l'ensemmence à partir d'une culture sur gélose. L'incubation est effectuée à 28°C pendant 72 heures.

Cet inoculum sert à ensemmencer un fermenteur de capacité de 100 litres contenant 50 litres du même milieu que précédemment.

Après 48 heures d'incubation à 28°C sous agitation, on ensemmence à partir de ce premier fermenteur

un deuxième fermenteur de 800 litres contenant 450 litres du milieu de culture suivant :

- distillers soluble : 25 g/l
- cerelose S.P.M. : 10 g/l
- huile de soja S10 : 5 g/l
- omyalite : 5 g/l
- sulfate d'ammonium : 2 g/l

Le PH est ajusté à 7 par de la soude et le milieu est stérilisé puis ensemmencé par 50 litres de la culture précédente.

L'incubation dure 93 heures à 26°C sous aération et sous agitation. Il est bien évident que le milieu de culture peut être modifié sans sortir du cadre de la présente invention. Ainsi, tout milieu de culture contenant une source hydrocarbonée, une source azotée, des éléments minéraux (phosphates, sels de fer, cobalt, magnésium, calcium) peut être utilisé sans le cadre de la présente invention.

Après fermentation, on obtient un milieu contenant 0,25 g/l de produit de formule (II).

La purification du produit de formule (II) est réalisée par passage sur une série de colonnes remplies de :

– résines polystyrène-divinylbenzène (Duolite S861)
– silicagel
– résine polyamide.

L'adsorption sur la résine Duolite est réalisée par mise en contact et évaporation d'une solution alcoolique de préférence méthanolique à 175 g d'extrait par litre d'alcool.

Le produit de formule (II) est élué par une solution de méthanol à 80-90 % et isolé par évaporation de l'alcool.

Ce produit est ensuite repris dans l'acétate d'éthyle et adsorbé sur silicagel puis élué par l'acétate d'éthyle d'où il est isolé par évaporation.

La dernière purification est réalisée par évaporation d'une solution méthanolique du produit en présence d'une résine polyamide, suivie d'une élution par une solution méthanol/eau de plus en plus concentrée en méthanol. On évapore le méthanol ce qui permet d'obtenir une solution aqueuse contenant le produit de formule (II).

Le produit de formule (II) est utilisé pour l'alimentation des animaux et plus particulièrement des animaux monogastriques. On l'utilise notamment pour l'alimentation du porcelet ou des volailles. L'activité facteur de croissance est au moins égale à celle des antibiotiques couramment utilisés dans ce domaine tout en ne présentant pratiquement aucun effet antibiotique.

La présente invention sera plus complètement décrite à l'aide des exemples suivants qui ne doivent en aucun cas être considérés comme limitatifs de l'invention.

## EXEMPLE 1

### 1.1 - Souche

Référence : Streptomyces CBS 473.89
Conservation : mélange de spores et de terre stérile, séché sous vide et gardé à +4°C en présence d'actigel

### 1.2 - Culture sur milieu gélosé

Récipient : tube de verre de L200 mm, diamètre 24 mm, bouché par un tampon de coton.
Remplissage : 22 ml

Milieu de culture :

| | |
|---|---|
| - amidon soluble DIFCO | : 10 g/l |
| - phosphate dipotassique PROLABO | : 1 g/l |
| - sulfate de magnésium, 7H20 PROLABO | : 1 g/l |
| - chlorure de sodium PROLABO | : 1 g/l |
| - sulfate d'ammonium PROLABO | : 2 g/l |
| - carbonate de calcium PROLABO | : 2 g/l |
| - gélose DIFCO | : 20 g/l |

PH non ajusté.
Stérilisation 20 minutes à 122°C ; pendant le refroidissement qui suit la stérilisation, les tubes sont disposés en position inclinée.
Ensemencement : à partir du mélange terre-spore (30 à 40mg/tube).
Incubation : 3-4 semaines à 26°C

1.3 - Culture inoculum (erlenmeyer de 2 litres)

Récipient : fiole erlenmeyer 2 litres avec 2 tubulures latérales dont l'une est munie d'un dispositif d'ensemencement. Fermeture par bouchon polyuréthane.
Remplissage : 400 ml

Milieu de culture :

| | |
|---|---|
| - peptone ORGANOTECHNIE | : 10 g/l |
| - extrait de levure SPRINGER | : 5 g/l |
| - glucose PROLABO | : 10 g/l |
| - chlorure de sodium PROLABO | : 5 g/l |
| - gélose PROLABO | : 1,5 g/l |

PH ajusté à 7,4 par 5 ml de soude 5 N.
Stérilisation : 20 minutes à 120°C, après stérilisation le PH est de 6,60.
Ensemencement : à partir d'une culture sur gélose pour 2 fioles.
Incubation : 72 heures à 28°C. Shaker 150 t/min : course 5 cm
PH en fin de culture voisin de 7,5.

1.4 - Culture inoculum (fermenteur de 100 litres)

Récipient : Fermenteur en acier inoxydable
Remplissage : 50 litres

Milieu de culture :

| | |
|---|---|
| - peptone ORGANOTECHNIE | : 10 g/l |
| - extrait de levure SPRINGER | : 5 g/l |
| - cerelose | : 10 g/l |
| - chlorure de sodium (sel fin épuré n° 2, Salins du Midi) | : 5 g/l |
| - gélose PROLABO | : 2 g/l |

PH avant stérilisation : 7,2
PH après stérilisation : 6,8
Stérilisation : 40 minutes à 122°C.
Ensemencement : à partir d'une culture inoculum en erlenmeyer (400 ml dans 50 litres soit 0,8 %).
Incubation : 45 heures à 28°C.
Agitation : 300 t :min ; aération 5 $m^3$/h
PH en fin de culture : 7,9
Antimousse consommé : 40 ml EMKAPYL (polypropylène glycol) dans la charge.

1.5 - Culture productrice (fermenteur de 800 litres)

Récipients : Fermenteur en acier inoxydable muni de 4 chicanes intérieures
Remplissage : 450 litres

Milieu de culture :

| | |
|---|---|
| - distillers solubles PRODULAC | : 25 g/l |
| - cerelose (société des produits du maïs) | : 10 g/l |
| - Huile de soja | : 5 g/l |
| - carbonate de calcium | : 5 g/l |
| - sulfate d'ammonium | : 2 g/l |

PH avant stérilisation : 7 (par 450 ml DE NaOH 10 N)
PH après stérilisation : 6,85
Stérilisation : 40 minutes à 122°C.
Ensemencement : 50 litres de la culture inoculum en fermenteur de 100 litres (50 litres dans 450 litres soit un peu plus de 10 %).
Incubation : 93 heures à 26°C
Agitation : turbine tournant à 250 t/min
Aération : 15 $m^3$/h sous 0,8 bar
PH en fin de culture : 7,8
Antimousse consommé : 100 ml d'EMKAPYL
Concentration du moût obtenu : 0,25 g de dérivé de formule (II)/1.

EXEMPLE 2 - EXTRACTION

Le pH du moût de fermentation (2 fermenteurs de 500 litres, soit en tout 1.000 litres de moût) est ajusté à 3 avec HCl 6N.

On ajoute 500 litres d'acétate d'éthyle et agite pendant 45 minutes puis centrifuge sur centrifugeuse WESTFALIA NA7 équipée pour la séparation liquide/liquide/solide.

La phase acétate d'éthyle, évaporée à sec sous pression réduite, donne une huile à laquelle on ajoute le résidu également huileux d'une seconde extraction effectuée dans les mêmes conditions avec 200 litres d'acétate d'éthyle.

On reprend cette huile dans 10 litres de méthanol. On ajoute 30 litres de cyclohexane, agite puis décante. Par évaporation de la phase la plus riche en méthanol on recueille 700 g de solide pulvérulent.

EXEMPLE 3 - PURIFICATION

On reprend les 700 g d'extrait obtenu à l'exemple 2 dans 4 litres de méthanol, on ajoute 4 litres de résine Duolite S861 et évapore à sec sous pression réduite. La résine enduite est ensuite déposée au sommet d'une colonne de 15 cm de diamètre renfermant 15 litres de la même résine vierge. Cette dernière ayant été préalablement conditionnée par un lavage au méthanol, suivi d'un rinçage à l'eau deminéralisée. On lave la colonne avec 20 litres de méthanol à 50 % puis 25 litres de méthanol à 70 %.

Les eaux de lavage sont éliminées. On élue ensuite en recueillant des fractions de 5 litres, avec 25 litres de méthanol à 80 % suivis de 60 litres de méthanol à 90 %. Un contrôle par CCM montre que les fractions 7 à 21 renferment le produit cherché. Par évaporation à sec sous pression réduite on recueille 372 g de produit.

La moitié de ce lot (186 g) est reprise par 800 ml d'acétate d'éthyle. A la solution obtenue, on ajoute 700 ml de silicagel de porosité 60 A et de granulométrie 40 à 63 µm (RHONE-POULENC LIMITED). On évapore à sec sous pression réduite et charge le solide pulvérulent dans une colonne en verre de 10 cm de diamètre sur une hauteur de 12 cm. On raccorde cette colonne à une colonne de même diamètre et de 50 cm de haut remplie de la même silice vierge. On élue par l'acétate d'éthyle pur au débit de 35 ml/min en recueillant des fractions de 500 ml. Un contrôle par CCM montre que le produit cherché est contenu dans les fractions 3 à 20 qui, regroupées et évaporées à sec abandonnent 104 g de solide.

On prélève 100 g de ce solide que l'on dissout dans 800 ml de méthanol. A la solution obtenue, on ajoute 700 ml de résine polyamide (polycaprolactame ou nylon 6) pour chromatographie de granulométrie 50 à 160 µm (MACHEREY-NAGEL). On évapore sous pression réduite et on charge la poudre sèche dans une colonne de 10 cm de diamètre et de 10 cm de hauteur. On adapte cette colonne à l'extrémité inférieure d'une colonne de 8 cm de diamètre et de 50 cm de haut remplie du même support vierge et sec.

On remplit de bas en haut avec du méthanol à 10 % pour chasser l'air puis on élue au débit de 35 ml/min en recueillant des fractions de 500 ml et en effectuant un step-gradient selon le tableau suivant :

| TENEUR EN MeOH | N° FRACTIONS DE 500 ml |
|---|---|
| 20 % | 1 à 3 |
| 30 % | 4 à 6 |
| 35 % | 7 à 10 |
| 40 % | 11 à 35 |
| 60 % | 36 à 76 |

En chromatographie couche mince, on détecte le produit dans les fractions 41 à 70 qui sont rassemblées. On évapore le méthanol sous pression réduite et on extrait la phase aqueuse résiduelle(PH neutre) par l'acétate d'éthyle. Par évaporation de la phase organique, on obtient une huile que l'on dilue dans 300 ml de méthanol. On coule cette solution très lentement dans 8 litres d'eau déminéralisée sous forte agitation. On obtient après séchage à l'étuve sous vide (40°C, 1 mm Hg 61 g de produit de formule (II).

ANALYSE PHYSICO-CHIMIQUES

On calcule pour $C_{37}H_{55}NO_{10}$ : C % = 65,95 ; H % = 8,23 ; N % = 2,08.
Trouvé : C % = 64,26 ; H % = 8,66 ; N % = 2,00 ; $H_2O$ = 1,66 %
$[\alpha]_D^{20}$ = + 30,9 + 1 (C = 0,5 ; méthanol)

| LONGUEUR D'ONDE MAX. nm | $E_{1\ cm}^{1\ \%}$ | ϵ |
|---|---|---|
| 289 | 538 | 36.200 |
| 233 | 762 | 51.200 |

CCM : chromatographie de silicagel MERCK $F_{254}$
épaisseur de la couche : 0,25 mm
Migration ascendante dans :
. dichloro-1,2 éthane 80 volumes
. méthanol 20 volumes
Révélation : UV 254 nm et vanilline sulfurique (bleu foncé)
$R_F$ = 0,35
– la structure du produit a été confirmée par résonance magnétique nucléaire à 400 Mégahertz dans le chloroforme deutéré
– le spectre infrarouge a été enregistré sur un spectromètre IR-Micolet 60 SxR, sous forme de pastilles de KBr.
Les bandes caractéristiques du spectre sont :

| | |
|---|---|
| 3425 $cm^{-1}$ | υ OH + υ NH |
| 3020 $cm^{-1}$ | υ CH (oléfine trans) |
| 2970 $cm^{-1}$ | $\upsilon_{as}$ $CH_3$ |
| 2930 $cm^{-1}$ | $\upsilon_{as}$ $CH_2$ |
| 2875 $cm^{-1}$ | $\upsilon_s$ $CH_3$ |
| 2820 $cm^{-1}$ | $\upsilon_s$ $CH_2$ |
| 3000-2450 $cm^{-1}$ | υ OH (acide) |
| 1690 $cm^{-1}$ | υ C = 0 (acide) |
| 1637 | υ C = C |
| 1620 $cm^{-1}$ | υ C = 0 (amide) |
| 1545 $cm^{-1}$ | δ NH |
| 1460 ; 1445 $cm^{-1}$ | δ $CH_2$ + $\delta_{as}CH_3$ |
| 1410 $cm^{-1}$ | δ OH |

| | |
|---|---|
| 1380 $cm^{-1}$ | $\delta_s CH_3$ |
| 1255 $cm^{-1}$ | υ C-O (acide) |
| 1095-1005 $cm^{-1}$ | υ C-O (éther + alcool) |
| 980 $cm^{-1}$ | W CH (CH=CH trans) |
| 935 $cm^{-1}$ | |

ACTIVITE ANTIBACTERIENNE DU COMPOSE DE FORMULE (II)

Description de la méthode de test utilisée

Les souches utilisées identifiées dans le tableau suivant sont décongelées au moment de l'emploi, puis sont diluées de façon à obtenir les concentrations suivantes :
- $10^7$ bactéries/ml pour les bactéries aérobies
- $10^6$ bactéries/ml pour les bactéries aérobies.

On réalise une suspension mère du produit de formule (II) à une concentration de 3000 mg/l dans l'eau. On dilue ensuite à l'eau de façon à obtenir les concentrations de 300 mg/l, 100 mg/l, 50 et 25.

Les milieux de culture sont les suivants :
- Mueller Hinton Agar (MHA) pour les cultures aérobies
- Mueller Hinton Agar (MHA) + 2 % de Fildes (MHA) pour les cultures anaérobies.

Mode opératoire :

On ajoute chaque dilution de produit à 20 ml de milieu de culture maintenus en surfusion à 45°C puis on coule en boîtes de Pétri. Après agitation douce les boîtes sont séchées ouvertes 1 heure à 37°C.

L'ensemencement est réalisé avec un inoculateur multipoint DENLEY qui dépose $10^{-3}$ ml de chaque inoculum par spot.

La lecture est effectuée après 18 heures d'incubation à 37°C pour les aérobies et 48 heures à 37°C pour les anaérobies.

La concentration minimale inhibitrice (CMI) est la plus petite concentration pour laquelle on n'observe pas de croissance.

ACTIVITE ANTIBACTERIENNE DU PRODUIT DE FORMULE (II)

| SOUCHES AEROBIES | CMI en mg/l |
|---|---|
| Staphylococcus aureus 209P | inactif à 300 |
| Staphylococcus aureus S $^{t}$A 1 | inactif à 300 |
| Staphylococcus aureus S $^{t}$A 2 | inactif à 300 |
| Staphylococcus aureus Weichbrodt | inactif à 300 |
| Staphylococcus epidermidis | inactif à 300 |
| Streptococcus faecalis | inactif à 300 |
| Streptococcus bovis 3 | inactif à 300 |
| Streptococcus bovis 5 | 25 |
| Streptococcus uberis 3 | 50 |
| Streptococcus uberis 8 | 100 |
| Escherichia coli INRA ECi | inactif à 300 |
| Escherichia coli DCO | inactif à 300 |
| Escherichia coli DC2 | 100 |
| Klebsiella pneumoniae Caroli | inactif à 300 |
| Enterobacter cloacae IP 6085 | inactif à 300 |
| Pseudomonas aeruginosa IP A 237 | inactif à 300 |
| Pasterella multocida IP | inactif à 300 |
| Proteus vulgaris IP A272 | inactif à 300 |
| Proteus mirabilis Villette | inactif à 300 |

| SOUCHES ANAEROBIES | CMI en mg/l |
|---|---|
| Clostridium perfringens IP 615 | inactif à 100 |
| Clostridium perfringens ATCC 13124 | inactif à 100 |
| Clostridium septicum | inactif à 100 |
| Bacteroides fragilis ATCC 25285 | inactif à 100 |
| Bacteroides fragilis 479R | inactif à 100 |
| Bacteroides thetaiotaomicron ATCC 29741 | inactif à 100 |
| Bacteroides melaninogenicus ATCC 15930 | inactif à 100 |

Utilisation du produit de formule II comme facteur de croissance

Cette expérience a permis, chez le porcelet sevré, de déterminer l'efficacité du produit de la formule II, comparée à celle de deux facteurs de croissance couramment utilisés : l'Embonate de Spiramycine (SPIRA 200, RHONE-POULENC) et la Tylosine (TYLAN 20, ELANCO).

Schéma expérimental :

L'expérience a comparé 5 traitements correspondant à la distribution d'un aliment porcelet témoin (aliment + acides aminés) ou du même mélange supplémenté en Spiramycine (40 ppm) essai comparatif 1, en Tylosine (40 ppm) essai comparatif 2 ou en composé de formule II (40 et 60 ppm) (essai 1 et 2).

TABLEAU 1

Composition centésimale et caractéristiques des régimes

| | Témoin | Compa 1 | Compa 2 | Essai 1 | Essai 2 |
|---|---|---|---|---|---|
| Maïs ..................... | 35 | 35 | 35 | 35 | 35 |
| Manioc ................... | 15 | 15 | 15 | 15 | 15 |
| Tourteau de soja .......... | 22,7 | 22,7 | 22,7 | 22,7 | 22,7 |
| Sucre .................... | 2 | 2 | 2 | 2 | 2 |
| Suif ..................... | 1,7 | 1,7 | 1,7 | 1,7 | 1,7 |
| Son fin .................. | 10 | 10 | 10 | 10 | 10 |
| Pois ..................... | 8,6 | 8,6 | 8,6 | 8,6 | 8,6 |
| C.M.V. ................... | 4 | 4 | 4 | 4 | 4 |
| Prémélange Témoin ......... | 1 | - | - | - | - |
| Prémélange Comp. 1 ........ | - | 1 | - | - | - |
| Prémélange Comp. 2 ........ | - | - | 1 | - | - |
| Prémélange Essai 1 ........ | - | - | - | 1 | - |
| Prémélange Essai 2 ........ | - | - | - | - | 1 |
| CARACTERISTIQUE CALCULEE | | | | | |
| Energie digestible (Kal/kg) | 3 200 | 3 200 | 3 200 | 3 200 | 3 200 |
| CARACTERISTIQUES ANALYSEES | | | | | |
| Matières azotées ........ % | 18,00 | | | | |
| Lysine .................. % | 1,14 | | | | |

TABLEAU 2

Composition centésimale du C.M.V.

| | |
|---|---|
| Phosphate bicalcique ....................... | 37,50 |
| Carbonate de chaux ......................... | 30,00 |
| Sel (Na Cl) ................................ | 10,00 |
| Oligo-éléments porcs 3042/Se ............... | 1,90 |
| Concentrat de choline 50 % ................. | 3,00 |
| Complexe ADEB Porcs 188 .................... | 0,50 |
| Support .................................... | 17,10 |

TABLEAU 3

Composition centésimale des prémélanges

| | Témoin | Comp. 1 | Comp. 2 | Essai 1 | Essai 2 |
|---|---|---|---|---|---|
| DL - Méthionine .......... | 11 | 11 | 11 | 11 | 11 |
| L-Lysine ................ | 20 | 20 | 20 | 20 | 20 |
| L-Thréonine ............. | 4 | 4 | 4 | 4 | 4 |
| Spira 200 ............... | - | 2 | - | - | - |
| Tylan 20 ................ | - | - | 20 | - | - |
| Formule (II) ............ | - | - | - | 0,4 | 0,6 |
| Amidon de maïs q.s.p 100 . | 65 | 63 | 45 | 64,6 | 64,4 |

Animaux

130 porcelets sevrés croisés Large-White et Landrace Belge ont été utilisés. L'expérience a débuté 2 semaines après le sevrage des animaux, soit à un âge moyen de 42 jours. Les porcelets d'un poids moyen de 10,7 kg ont été répartis en 13 blocs de 5 paires d'animaux de même poids.

Condition de granulation de l'aliment porcelet

Le lot d'aliment est granulé sur une presse à granuler du type KAHL 400 équipée d'une filière en acier inox d'épaisseur comprimante 35 mm, avec des trous de 2,5 mm de diamètre.

Avant la compression, la farine est conditionnée à la vapeur saturée 0,5 bars sur malaxeur continu.

Les températures relevées sont : 56°C pour la farine et 66°C pour les granulés à la sortie de la filière.

Les paramètres relevés sur la presse sont : 65 A (12 kw) au moteur principal et le débit de granulé est 700 kg/h.

Le refroidissement est assuré dans un refroidisseur vertical statique pendant 15 mn environ.

La dureté des granulés refroidis, mesurée à l'appareil Kahl après 72 h, est de 10 kg.

Alimentation

Les animaux ont reçu l'aliment expérimental à volonté sous forme de granulés de 2,5 mm de diamètre. La composition centésimale et les caractéristiques des régimes sont indiquées sans le tableau 1. Le tableau 2 indique la composition du Composé Minéral et Vitaminique (C.M.V.) et le tableau 3 celle des prémélanges d'additifs.

Contrôles

Les animaux ont été pesés en début, milieu et fin d'expérience. Les consommations ont été relevées toutes les semaines.

Résultats

Les résultats de consommation, croissance et d'efficacité alimentaire sont indiqués dans le tableau 4 pour la période 0-14 jours, dans le tableau 5 pour la période 14-28 jours et dans le tableau 6 pour la période 0-28 jours.

TABLEAU 4

Performance des animaux pendant la période 0-14 jours

| | Témoin | Comp. 1 | Comp. 2 | Essai 1 | Essai 2 |
|---|---|---|---|---|---|
| Poids initial (kg) ....... | 10,7 | 10,8 | 10,7 | 10,8 | 10,2 |
| Consommation (g/jour) .... | 614 | 668 | 619 | 625 | 599 |
| Gain de poids (g/jour) ... | 329 | 393 | 351 | 360 | 338 |
| Indice de consommation ... | 1,88 | 1,72 | 1,79 | 1,77 | 1,78 |
| Poids intermédiaire (kg) . | 15,3 | 16,3 | 15,6 | 15,8 | 15,3 |

TABLEAU 5

Performance des animaux pendant la période 14-28 jours

| | Témoin | Comp. 1 | Comp. 2 | Essai 1 | Essai 2 |
|---|---|---|---|---|---|
| Poids initial (kg) ....... | 15,3 | 16,3 | 15,6 | 15,8 | 15,3 |
| Consommation (g/jour) .... | 1031 | 1147 | 1033 | 1068 | 1026 |
| Gain de poids (g/jour) ... | 560 | 647 | 588 | 620 | 588 |
| Indice de consommation ... | 1,86 | 1,79 | 1,76 | 1,73 | 1,74 |
| Poids final (kg) ......... | 23,1 | 25,4 | 23,8 | 24,5 | 23,5 |

TABLEAU 6

Performance des animaux pendant la période 0-28 jours

| | Témoin | Comp. 1 | Comp. 2 | Essai 1 | Essai 2 |
|---|---|---|---|---|---|
| Poids initial (kg) ....... | 10,7 | 10,8 | 10,7 | 10,8 | 10,6 |
| Consommation (g/jour) .... | 822 | 908 | 826 | 847 | 812 |
| Gain de poids (g/jour) ... | 445 | 518 | 470 | 490 | 463 |
| Indice de consommation ... | 1,85 | 1,75 | 1,76 | 1,73 | 1,75 |
| Poids intermédiaire (kg) . | 23,1 | 25,4 | 23,8 | 24,5 | 23,5 |

Pendant la première période, on remarque déjà des tendances qui seront confirmées par la suite, à savoir: une augmentation de la consommation des animaux recevant le régime supplémenté avec la Spiramycine et une diminution de l'indice de consommation (rapport consommation/gain de poids) des animaux recevant les régimes supplémentés avec les facteurs de croissance.

Pendant la deuxième période (14-28 jours), on observe une différence significative entre régimes pour le critère "consommation".

La croissance des animaux est significativement améliorée par l'addition de Spiramycine au régime de base et est améliorée de la la même façon par l'addition des autres facteurs de croissance.

La période globale (0-28 jours) est la plus intéressante. Puisque plus longue, elle permet de déceler plus facilement les effets significatifs. La consommation et la croissance sont, pendant cette période, augmentées significativement par l'addition de Spiramycine au régime de base.

Le produit II à 40 ppm entraîne une amélioration de la croissance de 10 % par rapport au régime de base. Les performances des animaux nourris avec le régime contenant le produit II à 60 ppm sont un peu en retrait par rapport à celles observées avec le même produit à 40 ppm. L'indice de consommation est significativement amélioré quel que soit le facteur de croissance et quelle que soit la dose considérée.

Dans les conditions de notre expérience, si l'on considère la période globale :

– le produit II a amélioré de façon hautement significative l'indice de consommation des animaux. Cette amélioration est comparable à celle observée avec la Spiramycine et la Tylosine.

– le produit II au contraire de la Spiramycine n'a pas entraîné d'amélioration significative de la consommation. Pour ce critère, le produit est comparable à la Tylosine.

EXEMPLE 4

On reproduit l'exemple 3 en ce qui concerne l'utilisation du produit de formule II comme facteur de croissance à des doses de (10, 20 et 40 ppm, essais 3, 4 et 5) comparée à la spiramycine utilisée à une dose de 40 ppm.

Dans les tableaux 7, 8 et 9 sont indiqués respectivement la composition centésimale et les caractéristiques des régimes, la composition centésimale du CMV et la composition centésimale des prémélanges.

TABLEAU 7

Composition centésimales et caractéristiques des régimes

| | T | ESSAI 3 | ESSAI 4 | ESSAI 5 | SPIRA |
|---|---|---|---|---|---|
| Maïs .................... | | | 3,3 | | |
| Orge .................... | | | 28,2 | | |
| Son fin ................. | | | 9,1 | | |
| Suif .................... | | | 2,8 | | |
| Huile de maïs ........... | | | 1,0 | | |
| Manioc .................. | | | 15,0 | | |
| Sucre ................... | | | 2,0 | | |
| Tourtea de soja 48 ...... | | | 21,1 | | |
| Pois .................... | | | 10,0 | | |
| Farine de poisson 70 .... | | | 2,5 | | |
| C.M.V. .................. | | | 4,0 | | |
| Prémélange Témoin ....... | 1 | - | - | - | - |
| Prémélange Essai 3 ...... | - | 1 | - | - | - |
| Prémélange Essai 4 ...... | - | - | 1 | - | - |
| Prémélange Essai 5 ...... | - | - | - | 1 | - |
| Prémélange Spira ........ | - | - | - | - | 1 |
| <u>CARACTERISTIQUE</u> <u>CALCULEE</u> | | | | | |
| Energie nette (Kcal/kg) | | | 2 320 | | |
| Protéines brutes ...... % | | | 19,00 | | |
| Lysine totale ......... % | | | 1,19 | | |
| Lysine digestible ..... % | | | 1,03 | | |

TABLEAU 8

Composition centésimale du C.M.V.

| | |
|---|---|
| Phosphate bicalcique .................... | 35,75 |
| Carbonate de chaux ...................... | 25,00 |
| Sel (Na Cl) ............................ | 10,00 |
| Oligo-éléments (nouvelle formule) ........ | 2,00 |
| Complexe ADEB Porcelets (nouvelle fle) ... | 1,00 |
| Concentrat de choline 50 % .............. | 3,75 |
| Support (amidon) ........................ | 22,50 |

TABLEAU 9

Composition centésimale des prémélanges

| | Témoin | ESSAI 3 | ESSAI 4 | ESSAI 5 | SPIRA |
|---|---|---|---|---|---|
| DL-Méthonine ............. | 12,7 | 12,7 | 12,7 | 12,7 | 12,7 |
| L-Lysine HCl ............. | 11,7 | 11,7 | 11,7 | 11,7 | 11,7 |
| L-Thréonine .............. | 3,1 | 3,1 | 3,1 | 3,1 | 3,1 |
| L-Tryptophane ............ | 1,0 | 1,0 | 1,0 | 1,0 | 1,0 |
| Composé formule II à 90 % . | - | 0,11 | 0,22 | 0,44 | - |
| Spiramycine .............. | - | - | - | - | 2,0 |
| Amidon de maïs ........... | 71,5 | 71,39 | 71,28 | 71,06 | 69,5 |

Le protocole expérimental, condition de granulation, animaux, alimentation, contrôles sont les mêmes que dans l'exemple 1.

Les résultats sont indiqués dans le tableau 10 pour la période 0-14 jours et le tableau 11 pour la période de 14-28 jours et le tableau 12 pour la période 0-28 jours.

TABLEAU 10

Performance des animaux pendant la période 0-14 jours

| | T | ESSAI 3 | ESSAI 4 | ESSAI 5 | SPIRA |
|---|---|---|---|---|---|
| Poids initial (kg) | 11,5 | 11,5 | 11,5 | 11,5 | 11,4 |
| Consommation (g/j) | 645 | 694 | 689 | 701 | 715 |
| Gain de poids (g/j) | 368 | 418 | 430 | 416 | 423 |
| Indice de consommation | 1,76 | 1,70 | 1,61 | 1,70 | 1,70 |

TABLEAU 11

Performances des animaux pendant la période 14-28 jours

| | T | ESSAI 3 | ESSAI 4 | ESSAI 5 | SPIRA |
|---|---|---|---|---|---|
| Consommation (g/j) | 1059 | 1133 | 1164 | 1099 | 1130 |
| Gain de poids (g/j) | 579 | 648 | 664 | 648 | 640 |
| Indice de consommation | 1,84 | 1,75 | 1,75 | 1,70 | 1,77 |
| Poids final (kg) | 24,7 | 26,4 | 26,8 | 26,4 | 26,3 |

TABLEAU 12

Performances des animaux pendant la période 0-28 jours

| | T | ESSAI 3 | ESSAI 4 | ESSAI 5 | SPIRA |
|---|---|---|---|---|---|
| Poids initial (kg) | 11,5 | 11,5 | 11,5 | 11,5 | 11,4 |
| Consommation (g/j) | 852 | 914 | 927 | 900 | 923 |
| Gain de poids (g/j) | 474 | 533 | 547 | 532 | 532 |
| Indice de consommation | 1,80 | 1,72 | 1,69 | 1,70 | 1,73 |
| Poids final (kg) | 24,7 | 26,4 | 26,8 | 26,4 | 26,3 |

## Revendications

**1** - Composé de formule (II) non antiobiotique et ses sels

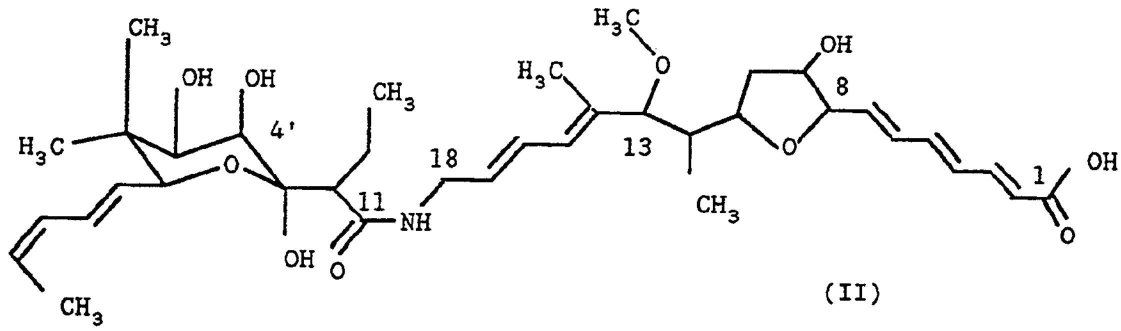

(II)

**2** - Sels du composé de formule (II) choisis parmi les sels alcalins, alcalino-terreux et azotés.

**3** - Préparation du composé de formule (II) selon la revendication 1 caractérisée en ce qu'on réalise une fermentation aérobie d'un microorganisme du genre Streptomyces déposé à la C.B.S. sous le numéro 473.89 ou d'un de ses mutants.

**4** - Utilisation du composé de formule (II) comme facteur de croissance pour l'alimentation des animaux monogastriques à des doses pharmacologiquement acceptables.

**5** - Utilisation du composé de formule (II) comme facteur de croissance pour l'alimentation du porcelet et/ou des volailles.

Office européen des brevets

# RAPPORT DE RECHERCHE EUROPEENNE

Numero de la demande

EP 91 40 0294

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | US-A-4 476 139 (A.J. KEMPF et al.)<br>* En entier * | 1-5 | C 07 H 7/02<br>C 12 P 19/02<br>A 23 K 1/17 |
| A | EP-A-0 252 380 (AMERICAN CYANAMID)<br>* Revendications 1-30 * & US-A-4 705 688; & JP-A-4 753 798 (Cat. D) | 1-5 | |
| A | THE JOURNAL OF ANITBIOTICS, vol. XLI, no. 10, octobre 1988, pages 1511-1514; G.T. CARTER et al.: "LL-E19020alpha and beta, novel growth promoting agents: isolation, characterization and structures"<br>* En entier * | 1-3 | |
| A | THE JOURNAL OF ANTIBIOTICS, vol. 41, octobre 1988, pages 1300-1315; J.E. HOCHLOWSKI et al.: "Phenelfamycins, a novel complex of elfamycin-type antibiotics. II. Isolation and structure determination"<br>* Page 1300, abrégé * | 1-3 | |

DOMAINES TECHNIQUES RECHERCHES (Int. Cl.5)

C 07 H 7/00
C 12 P 19/00
A 23 K 1/00

Le présent rapport a été établi pour toutes les revendications

| Lieu de la recherche | Date d'achèvement de la recherche | Examinateur |
|---|---|---|
| LA HAYE | 23-04-1991 | BRENNAN J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)